# EUROPÄISCHE PATENTSCHRIFT

(11) **EP 0 660 727 B1**
(45) Veröffentlichungstag und Bekanntmachung des Hinweises auf die Patenterteilung: **17.12.1997**
(21) Anmeldenummer: 93917717.6
(22) Anmeldetag: 31.07.1993
(51) Int. Cl.: A61M 5/14, A61M 39/00

(54) **SICHERHEITSVERBINDER FÜR DIE INFUSIONSTHERAPIE**
SAFETY CONNECTOR FOR INFUSION THERAPY
RACCORD DE SECURITE POUR LA THERAPIE PAR PERFUSION

(30) Priorität: 18.09.1992 DE 4231276
(43) Veröffentlichungstag der Anmeldung: 05.07.1995
(73) Patentinhaber: Haindl, Hans, Dr., D-30974 Wennigsen (DE)
(72) Erfinder: Haindl, Hans, Dr., D-30974 Wennigsen (DE)
(74) Vertreter: Leine, Sigurd, Dipl.-Ing.
(86) Internationale Anmeldenummer: EP9302049
(87) Internationale Veröffentlichungsnummer: WO9406489

(56) Entgegenhaltungen:
- US-A- 4 604 093

## Beschreibung

Die Erfindung betrifft einen Sicherheitsverbinder der im Oberbegriff des Anspruchs 1 genannten Art für die Infusionstherapie zur Zuführung mehrerer Infusionsleitungen.

Durch DE 40 04 134 A1 ist ein Sicherheitsverbinder der betreffenden Art bekannt. Dieser weist ein von der Infusionsflüssigkeit durchströmtes Gehäuse mit mehreren jeweils separat verschließbaren Zugängen sowie einem Abgang für die zusammengeführte Flüssigkeit auf. Die Zugänge sind abgewinkelt und münden in einer gemeinsamen Ebene. Ein im Querschnitt im wesentlichen winkelförmiges elastisches Dichtelement ist mit seinem im wesentlichen senkrecht zu der genannten Ebene verlaufenden Schenkel gehalten, während der andere Schenkel mit Federdruck in der genannten Fläche gegen die Mündungen der Zugänge drückt und diese verschließt. Auf diese Weise ist für jeden Zugang ein Rückschlagventil gebildet, das den Eintritt einer Injektionsflüssigkeit aus einem Zugang in die Mündung eines anderen Zugangs verhindert.

Dieser bekannte Sicherheitsverbinder hat mehrere Nachteile. Zunächst ist er äußerst kompliziert im Aufbau. Das im Querschnitt winkelförmige Dichtelement muß genau und dicht eingespannt sein. Darüber hinaus muß es mit seinem dichtenden Schenkel in der Ebene, in der die Zugänge münden, dicht an der Mündungsfläche anliegen. Da dabei die Anlagekraft durch eine Schwenkbewegung des dichtenden Schenkels des Dichtungselements erzeugt wird, ist es außerordentlich schwierig, eine vollständige Dichtung rund um den Mündungsbereich eines Zugangs und dies auch für alle Zugänge zu erreichen. Dies kann außerdem nur dann der Fall sein, wenn die Anlagekraft verhältnismäßig groß ist. Dann muß aber auch der öffnungsdruck verhältnismäßig groß sein. Aus diesem Grund ist dieser bekannte Sicherheitsverbinder für Schwerkraftinfusionseinrichtungen nicht geeignet.

Durch DE-GM 74 22 657 ist ein Mehrfachverbindungsstück für Leitungen medizinischer Geräte mit einem seitlichen Innenkonus zum Ansetzen eines Verbindungsstücks mit Außenkonus und einem im Bereich des seitlichen Anschlußstücks angeordneten elastischen, im Normalzustand an der Innenwandung des Mehrfachverbindungsstücks anliegenden Schlauchstück bekannt. Der Innenkonus besitzt eine solche Weite, daß die Stirnseite des Außenkonus des Verbindungsstücks in eingesteckter Lage das Schlauchstück aus seiner abdichtenden Lage in die Offenstellung drückt. Das elastische Schlauchstück soll dabei auch als Rückschlagventil wirken und sich zeitweilig öffnen, wenn mit Hilfe einer Injektionsspritze ein entsprechend hoher Injektionsdruck erzeugt wird. Das Zuführen einer Injektionslösung setzt also voraus, daß ein entsprechend hoher Druck in der Injektionsleitung erzeugt werden kann. Damit ist der Anwendungsbereich dieses bekannten Verbinders außerordentlich begrenzt, jedenfalls nicht für Schwerkraftinfusionsgeräte geeignet. Ist das Verbindungsstück außerdem als Mehrfachverbindungsstück ausgebildet, so hat es einen komplizierten Aufbau, bei dem an einem zentralen, längeren Rohrstück mehrere Anschlüsse für mehrere Infusionsleitungen vorgesehen sind. Dadurch ergibt sich ein kompliziertes, unübersichtliches und langes Gebilde.

Der Erfindung liegt die Aufgabe zugrunde, einen Sicherheitsverbinder der im Oberbegriff des Anspruchs 1 genannten Art für die Infusionstherapie zur Zusammenführung mehrerer Infusionsleitungen zu schaffen, der einfach im Aufbau, preiswert, übersichtlich und insbesondere auch für die Verwendung bei Schwerkraftinfusionen geeignet ist.

Die der Erfindung zugrundeliegende Aufgabe wird durch die im Kennzeichen des Anspruchs 1 angegebene Lehre gelöst.

Der Grundgedanke der Erfindung besteht darin, die Überdruckventile für die einzelnen Anschlußkanäle in einer zylindrischen Fläche anzuordnen. Solche zylindrischen Flächen lassen sich grundsätzlich einfach und genau herstellen. Zu diesem Zweck ist die Kammer, die zwischen den Anschlußkanälen und dem Abflußkanal angeordnet ist, durch eine im wesentlichen zylindrische Innenwandung des Gehäuses umgrenzt. Die Anschlußleitungen münden in dieser zylindrischen Innenwandung des Gehäuses. Der elastische Ventilkörper ist ringförmig ausgebildet und weist eine zylindrische Außenwandung auf, die an der die Kammer umschließenden Innenwandung des Gehäuses anliegt, in der auch die Anschlußkanäle münden. Sowohl die genannte Innenwandung des Gehäuses als auch die zylindrische Außenwandung des ringförmigen Ventilkörpers lassen sich mit hoher Genauigkeit fertigen, so daß schon bei sehr niedrigen Anlagekräften ein dichter Verschluß der so gebildeten Rückschlagventile gewährleistet ist. Dabei ist gleichzeitig die Anlagekraft sehr genau bestimmbar durch den Umfang des ringförmigen Ventilkörpers in bezug zu dem Umfang der zylindrischen Innenwandung des Gehäuses. Außerdem ist die Anlagekraft durch die elastische Nachgiebigkeit des ringförmigen Ventilkörpers in Umfangsrichtung bestimmt. Damit geht also die gesamte Umfangslänge des ringförmigen Ventilkörpers in die Anlagekraft ein, und da diese sehr genau bestimmbar ist, ist auch die Anlage und damit Abdichtkraft sehr genau bestimmbar. Dies gilt insbesondere auch für sehr geringe Drücke bis herab zu wenigen Hektopascal, so daß der erfindungsgemäße Sicherheitsverbinder aufgrund der Ausbildung der Ventile besonders gut für die Zusammenführung mehrerer Schwerkraftinfusionsleitungen geeignet ist.

Gemäß einer Weiterbildung der Erfindung ist die axiale Ausdehnung der zylindrischen Wandung des Ventilkörpers kleiner als die axiale Ausdehnung der zylindrischen Innenwandung des Gehäuses. Dadurch ist eine freie Beweglichkeit des Ventilkörpers gewährleistet, die insbesondere bei sehr geringen öffnungsdrücken von besonderer Bedeutung ist.

Bei dieser Weiterbildung ist es zweckmäßig, wenn das Gehäuse in die Kammer ragende Vorsprünge aufweist, die den ringförmigen Ventilkörper in seiner axialen Lage halten. Die Vorsprünge sind dabei zweckmäßigerweise so angeordnet und bemessen, daß an den axialen Rändern des ringförmigen Ventilkörpers ein Raum zum Durchtritt von Infusionsflüssigkeit gebildet ist.

Das Gehäuse ist zweckmäßigerweise scheibenförmig, und der Abflußkanal ist axial, vorzugsweise koaxial dazu angeordnet. Dadurch ergibt sich ein übersichtliches, axialsymmetrisches Äußeres, das insbesondere dann von Vorteil ist, wenn sich gemäß einer Ausführungsform dieser Weiterbildung axial gegenüber dem Abflußkanal ein Verbindungskanal befindet, der zum Verbinden mehrerer erfindungsgemäßer Sicherheitsverbinder geeignet ist, aber auch zur Entlüftung verwendet werden kann.

Eine Weiterbildung der Erfindung besteht darin, daß die Kammer ringförmig ist. Dadurch ist der Sammelraum zwischen den einzelnen Anschlußkanälen und dem Abflußkanal sehr gering, so daß so kein unnützer toter Raum gebildet ist, der zu dem Nachteil führen kann, daß sich darin Infusionsflüssigkeit einer Art sammelt, die erst bei Zuführung einer Infusionsflüssigkeit einer anderen Art weiterbefördert wird und somit verspätet zur Wirkung kommt.

Der ringförmige Ventilkörper hat zweckmäßigerweise die Form eines kurzen Schlauchabschnitts. Er läßt sich also ganz einfach durch Ablängen kurzer Stücke eines elastischen Schlauchs herstellen. Zweckmäßig ist es auch, daß die Innenwandung des Schlauchabschnitts im Querschnitt ballig ist. Das führt dazu, daß die elastische Nachgiebigkeit des zylindrischen Ventilkörpers zu den Rändern hin zunimmt, andererseits zu benachbarten Mündungen von Anschlußkanälen hin größer ist. Dadurch wird die Gefahr eines Übergangs einer Infusionsflüssigkeit aus der Mündung eines Zuführungskanals in die Mündung eines anderen Zuführungskanals verringert.

Dem gleichen Zweck dient eine Weiterbildung der Erfindung, gemäß der jeweils zwischen den Mündungen der Anschlußkanäle in der die Kammer umschließenden zylindrischen Innenwandung des Gehäuses Ausnehmungen angeordnet sind, die mit der Kammer in Verbindung stehen. Diese bewirken, daß Infusionsflüssigkeit, die aus einer Mündung eines Anschlußkanals in Richtung auf die Mündung eines anderen Anschlußkanals zu fließen sucht, zwangsläufig in diese Ausnehmungen gelangt und damit auch in die sammelnde zentrale Kammer. Mit anderen Worten, die Ausnehmungen bewirken praktisch eine Trennung der einzelnen Überdruckventile voneinander. Die Ausnehmungen haben zweckmäßigerweise die Form von axialen Bohrungen, die sich über die axialen Ränder der zylindrischen Außenwandung des ringförmigen Ventilkörpers hinaus erstrecken.

Nach der erfindungsgemäßen Lehre soll die zylindrische Innenwandung des Gehäuses, die die Kammer umgrenzt, im wesentlichen zylindrisch sein. Das bedeutet, daß natürlich dem Fachmann geläufige und das Wesen der Erfindung berücksichtigende Abweichungen möglich sind. So kann die Innenwandung auch in geringem Maß kegelig oder auch ballig sein. Eine solche Balligkeit ist zwar schwieriger herzustellen, sie führt aber beispielsweise zu dem gleichen Vorteil wie die Balligkeit der im Querschnitt innenliegenden Fläche eines im wesentlichen durch einen Schlauchabschnitt gebildeten Ventilkörpers. Die Balligkeit erhöht die Anlagekräfte im zentralen Bereich der Mündungen der Anschlußkanäle, so daß dadurch ein Austausch von Infusionsflüssigkeit zwischen den Anschlußkanälen behindert und der Abfluß zu den Rändern des Ventilkörpers und damit in die Kammer begünstigt wird.

Anhand der Zeichnung soll die Erfindung an einem Ausführungsbeispiel näher erläutert werden.
- Fig. 1: zeigt eine Draufsicht auf ein Ausführungsbeispiel eines Sicherheitsverbinders gemäß der Erfindung,
- Fig. 2: ist ein vergrößerter Schnitt II-II durch Fig. 1,
- Fig. 3: ist ein Schnitt III-III durch Fig. 2 im Maßstab von Fig. 1 und
- Fig. 4: ist ein vergrößerter Ausschnitt IV aus Fig. 3.

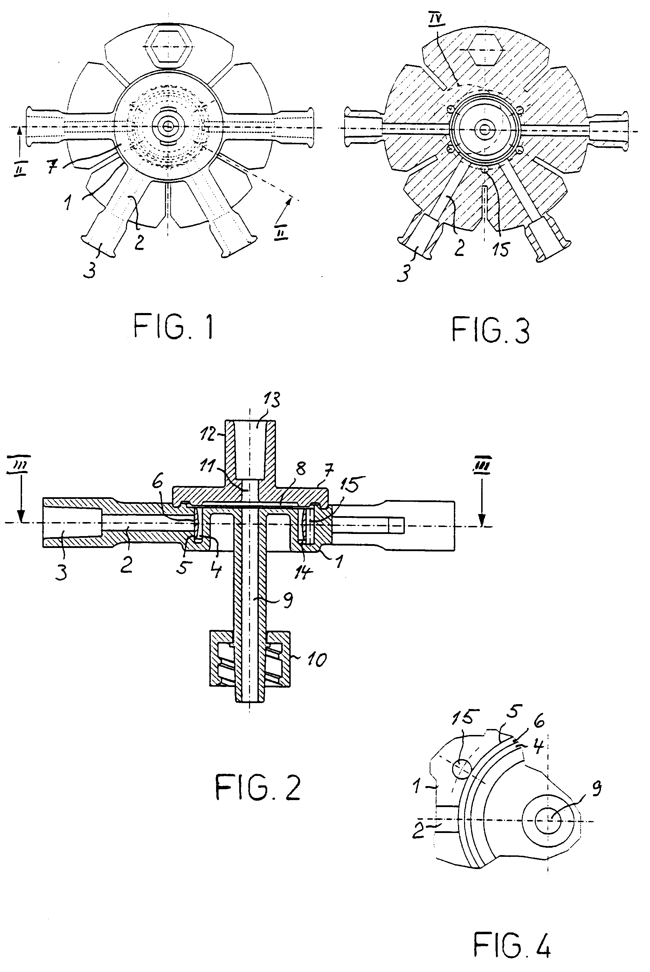

Wie aus den Fig. 1 bis 3 ersichtlich, weist der erfindungsgemäße Sicherheitsverbinder ein im wesentlichen scheibenförmiges Gehäuse 1 auf, in dem sich radial oder sternförmig angeordnete Anschlußkanäle 2 befinden, die sich nach außen in Luer-Lok-Anschlüsse 3 fortsetzen und innen in eine ringförmige Kammer 4 münden, die außen durch eine zylindrische Innenwandung 5 des Gehäuses 1 umgrenzt ist, an der mit einer vorgegebenen, in der Regel sehr geringen Anlagekraft ein Ventilkörper in Form eines kurzen Schlauchabschnitts 6 mit einer zylindrischen Außenfläche dicht anliegt.

Die ringförmige Kammer 4 ist durch ein scheibenförmiges Gehäuseteil 7 verschlossen, das mit dem Gehäuse 1 verklebt ist. Die ringförmige Kammer 4 steht über einen schmalen, scheibenförmigen Raum 8 mit einer Abflußleitung 9 in Verbindung, an deren Ende sich ein Anschlußstück 10 befindet. Außerdem steht der scheibenförmige Raum 8 mit einem Verbindungskanal 11 in Verbindung, an den sich ein Verbindungsstück 12 mit einen Innenkonus 13 anschließt, mit dessen Hilfe ein weiterer und gleicher Sicherheitsverbinder anschließbar ist, so daß die Zahl der zuführbaren Infusionsleitungen vervielfacht wird. Das Anschlußstück 12 kann auch ganz einfach verschlossen und nur zum Zweck von Entlüftungen geöffnet werden.

Wie aus Fig. 2 ersichtlich, ist die Innenfläche des kurzen Schlauchabschnitts 6, der den Ventilkörper bildet, im Querschnitt ballig ausgebildet. Dadurch ist die Anlagekraft im mittleren Bereich größer als an den Rändern. Bei einem Überdruck entweicht somit die Infusionsflüssigkeit vorwiegend über die Ränder in die Kammer 4, so daß die Gefahr verringert ist, daß Infusionsflüssigkeit aus einer Mündung eines der Anschlußkanäle 2 in die Mündung eines benachbarten Anschlußkanals 2 eintritt.

Wie aus Fig. 2 weiterhin ersichtlich, weist das Gehäuse 1 zu der ringförmigen Kammer 4 hin und anliegend an den Rändern des kurzen Schlauchabschnitts 6 Vorsprünge 14 auf, die den kurzen Schlauchabschnitt 6 in ausgerichteter Lage zu den Mündungen der Anschlußkanäle 2 halten.

Aus Fig. 3 und insbesondere aus dem vergrößerten Ausschnitt IV aus Fig. 3 in Fig. 4 ist ersichtlich, daß zwischen den Mündungen benachbarter Anschlußkanäle in der zylindrischen Innenwandung des Gehäuses 1 Ausnehmungen 15 angeordnet sind, die sich über die Ränder des kurzen Schlauchabschnitts 6 hinaus erstrecken, wie das aus Fig. 2 ersichtlich ist. Auf diese Weise ist zwischen den Mündungen der Anschlußkanäle 2 ein Abfluß gebildet, durch den in die Kammer 4 Infusionsflüssigkeit abfließen kann, die durch den Dichtungsbereich zwischen der Innenwandung 5 des Gehäuses 1 und der Außenwandung des kurzen Schlauchabschnitts 6 in Richtung auf eine benachbarte Mündung eines Anschlußkanals 2 vorgedrungen ist. Sie kann so nicht die Mündung des benachbarten Anschlußkanals erreichen und in diesen eindringen. Eine unerwünschte Mischung von Infusionsflüssigkeiten durch Rückfluß in eine andere Infusionsleitung ist somit nicht möglich.

## Patentansprüche

1. Sicherheitsverbinder für die Infusionstherapie zur Zuführung mehrerer Infusionsleitungen,
mit einem Gehäuse (1),
mit einer in dem Gehäuse (1) angeordneten Kammer (4), in die mehrere Anschlußkanäle (1) zum Anschluß von Infusionsleitungen münden und die einen Abflußkanal (9) zum Anschluß einer Abflußleitung aufweist, und
mit einem elastischen Ventilkörper, der in der Kammer (4) angeordnet ist und mit einer sich über alle Mündungen der Anschlußkanäle (2) hinweg erstreckenden Wandung diese Mündungen verschließt, derart, daß durch einen gemeinsamen Ventilkörper Rückschlagventile für alle Anschlußkanäle (2) gebildet sind,
**dadurch gekennzeichnet**,
daß die Kammer (4) durch eine im wesentlichen zylindrische Innenwandung (5) des Gehäuses (1) umgrenzt ist,
daß die Anschlußkanäle (2) in der zylindrischen Innenwandung (5) des Gehäuses (1) münden,
daß der elastische Ventilkörper ringförmig ist und eine zylindrische Außenwandung aufweist und
daß die zylindrische Außenwandung des Ventilkörpers an der die Kammer (4) umschließenden zylindrischen Innenwandung (5) des Gehäuses (1) anliegt.

2. Sicherheitsverbinder nach Anspruch 1, **dadurch gekennzeichnet**, daß die axiale Ausdehnung der zylindrischen Außenwandung des Ventilkörpers kleiner ist als die axiale Ausdehnung der zylindrischen Innenwandung (5) des Gehäuses (1).

3. Sicherheitsverbinder nach Anspruch 2, **dadurch gekennzeichnet**, daß das Gehäuse (1) in die Kammer (4) ragende Vorsprünge (14) aufweist, die den ringförmigen Ventilkörper in seiner axialen Lage halten.

4. Sicherheitsverbinder nach Anspruch 3, **dadurch gekennzeichnet**, daß die Vorsprünge (14) so angeordnet und bemessen sind, daß an den axialen Rändern des ringförmigen Ventilkörpers ein Raum zum Durchtritt für Infusionsflüssigkeit gebildet ist.

5. Sicherheitsverbinder nach Anspruch 1, **dadurch gekennzeichnet**, daß das Gehäuse (1) scheibenförmig und der Abflußkanal (9) axial, vorzugsweise koaxial dazu angeordnet ist.

6. Sicherheitsverbinder nach Anspruch 5**, dadurch gekennzeichnet**, daß dem Abflußkanal (9) axial gegenüber ein Verbindungskanal (11) angeordnet ist.

7. Sicherheitsverbinder nach Anspruch 1, **dadurch gekennzeichnet**, daß die Kammer (4) ringförmig ist.

8. Sicherheitsverbinder nach Anspruch 1, **dadurch gekennzeichnet**, daß der ringförmige Ventilkörper die Form eines kurzen Schlauchabschnitts (6) hat.

9. Sicherheitsverbinder nach Anspruch 8, **dadurch gekennzeichnet**, daß die Innenwandung des kurzen Schlauchabschnitts (6) im Querschnitt ballig ist.

10. Sicherheitsverbinder nach Anspruch 1, **dadurch gekennzeichnet**, daß jeweils zwischen den Mündungen der Anschlußkanäle (2) in der die Kammer (4) umschließenden Innenwandung (5) des Gehäuses (1) Ausnehmungen angeordnet sind, die mit der Kammer (4) in Verbindung stehen.

11. Sicherheitsverbinder nach Anspruch 10, **dadurch gekennzeichnet**, daß die Ausnehmungen die Form von axialen Bohrungen (15) haben, die sich über die axialen Ränder der zylindrischen Außenwandung des ringförmigen Ventilkörpers hinaus erstrecken.

## Claims

1. Safety connector for infusion therapy for the supply of several infusion tubes, comprising a housing (1), a chamber (4), which is situated in the housing (1), into which open several connection channels (2) for connection to infusion tubes and which has an outlet channel (9) for the connection of an outlet tube, and further comprising an elastic valve body which is arranged in the chamber (4) and has a wall extending across all outlets of the connection channels (2) by means of which it closes them such that non-return valves for all connection channels (2) are formed by a common valve body,
characterised in that the chamber (4) is surrounded by a substantially cylindrical inner wall (5) of the housing (1), that the connection channels (2) open into the cylindrical inner wall (5) of the housing (1), that the elastic valve body is ring-shaped and comprises a cylindrical outer wall, and that the cylindrical outer wall of the valve body bears on to the cylindrical inner wall (5) of the housing (1) surrounding the chamber (4).

2. Safety connector according to claim 1, characterised in that the axial dimension of the cylindrical outer wall of the valve body is smaller than the axial dimension of the cylindrical inner wall (5) of the housing (1).

3. Safety connector according to claim 2, characterised in that the housing (1) comprises projections (14) which extend into the chamber (4) and which keep the ring-shaped valve body in its axial position.

4. Safety connector according to claim 3, characterised in that the projections (14) are so arranged and dimensioned, that a space for the passage of infusion liquid is formed at the axial edges of the ring-shaped valve body.

5. Safety connector according to claim 1, characterised in that the housing (1) is disk-shaped and the outlet channel (9) is arranged axially, preferably coaxially thereto.

6. Safety connector according to claim 5, characterised in that the outlet channel (9) is arranged axially with respect to the connection channel (11).

7. Safety connector according to claim 1, characterised in that the chamber (4) is ring-shaped.

8. Safety connector according to claim 1, characterised in that the ring-shaped valve body has the shape of a short tube section (6).

9. Safety connector according to claim 8, characterised in that the inner wall of the short tube section (6) is bulging in cross-section.

10. Safety connector according to claim 1, characterised in that recesses are provided between the outlet openings of the connection channels (2) in the inner wall (5) of the housing (1) which surrounds the chamber (4), the recesses being in connection with the chamber (4).

11. Safety connector according to claim 10, characterised in that the recesses have the shape of axial bores (15) which extend beyond the axial edges of the cylindrical outer wall of the ring-shaped valve body.

## Revendications

1. Raccord de sécurité pour la thérapie par perfusion pour l'amenée de plusieurs conduites de perfusion,
avec un carter (1),
avec une chambre (4) disposée dans le carter (1), dans laquelle débouchent plusieurs canaux de raccordement (1) pour le branchement de conduites de raccordement et qui présente un canal d'évacuation (9) pour le raccordement d'une conduite d'évacuation, et
avec un corps de soupape élastique qui est disposé dans la chambre (4) et qui obture, par une paroi s'étendant sur tous les orifices des canaux de raccordement (2), ces orifices de telle sorte que des clapets antiretour pour tous les canaux de raccordement (2) sont formés par un corps de soupape commun,
caractérisé en ce,
que la chambre (4) est délimitée par une paroi interne (5) du carter (1) essentiellement cylindrique,
que les canaux de raccordement (2) débouchent dans la paroi interne (5) cylindrique du carter (1),
que le corps de soupape élastique est de forme annulaire et présente une paroi externe cylindrique et
la paroi externe cylindrique du corps de soupape est en appui sur la paroi interne cylindrique (5) du carter (1) entourant la chambre (4).

2. Raccord de sécurité selon la revendication 1,
caractérisé en ce que la dimension axiale de la paroi externe cylindrique du corps de soupape est plus petite que la dimension axiale de la paroi interne (5) cylindrique du carter (1).

3. Raccord de sécurité selon la revendication 2,
caractérisé en ce que le carter (1) présente des protubérances (14) faisant saillie dans la chambre (4) qui maintiennent le corps de soupape de forme annulaire dans sa position axiale.

4. Raccord de sécurité selon la revendication 3,
caractérisé en ce que les protubérances (14) sont disposées et dimensionnées de telle sorte qu'un espace pour le passage du liquide de perfusion est formé sur les bords axiaux du corps de soupape de forme annulaire.

5. Raccord de sécurité selon la revendication 1,
caractérisé en ce que le carter (1) est en forme de disque et le canal d'évacuation (9) lui est disposé axial, de préférence coaxial.

6. Raccord de sécurité selon la revendication 5,
caractérisé en ce qu'un canal de liaison (11) est adjoint axialement à l'opposé du canal d'évacuation (9).

7. Raccord de sécurité selon la revendication 1,
caractérisé en ce que la chambre (4) est de forme annulaire.

8. Raccord de sécurité selon la revendication 1,
caractérisé en ce que le corps de soupape de forme annulaire a la forme d'un segment de tuyau flexible court (6).

9. Raccord de sécurité selon la revendication 8,
caractérisé en ce que la paroi interne du segment de tuyau flexible court (6) est convexe en section transversale.

10. Raccord de sécurité selon la revendication 1,
caractérisé en ce que des évidements, qui sont en liaison avec la chambre (4), sont ménagés dans la paroi interne (5) du carter (1) entourant la chambre (4) respectivement entre les orifices des canaux de raccordement (2).

11. Raccord de sécurité selon la revendication 10,
caractérisé en ce que les évidements ont la forme d'alésages axiaux (15) qui s'étendent au-dessus des bords axiaux de la paroi externe cylindrique du corps de soupape de forme annulaire.
